# EUROPEAN PATENT SPECIFICATION

(11) **EP 0 477 209 B1**
(45) Date of publication and mention of the grant of the patent: **27.04.1994**
(21) Application number: 90908497.2
(22) Date of filing: 23.05.1990
(51) Int. Cl.: A61K 9/30, A61K 6/08, A61L 27/00

(54) **A PREPARATION FOR THE TREATMENT OF FUNGAL INFECTIONS**
ZUBEREITUNG ZUR BEHANDLUNG VON PILZINFEKTIONEN
PREPARATION POUR LE TRAITEMENT D'INFECTIONS FONGIQUES

(30) Priority: 25.05.1989 DK 2542/89
(43) Date of publication of application: 01.04.1992
(73) Proprietor: A/S DUMEX (DUMEX LTD.), 2300 Copenhagen S (DK)
(72) Inventor: NORLING, Tomas, DK-2100 Copenhagen (DK)
(74) Representative: Kyed, Iver
(86) International application number: DK9000129
(87) International publication number: WO9014076

(56) References cited:
- EP-A- 0 250 187
- WO-A-87/02580
- GB-A- 1 603 899
- US-A- 3 476 854
- Dialog Information Services, file 351: WPI, WPI Acc No. 86-003018/01 & JP-A-60228412 (Terumo Corp.) 13 November 1985, Abstrtact
- Dialog Information Services, file 351: WPI, WPI Acc. No. 88-209883/30 & JP-A-63146972 (Sekisui Chem Ind. K.K.) 18 June 1988, Abstract

## Description

### Field of the Invention

The present invention concerns a denture lacquer for the treatment of fungal infections in the oral cavity of dentures wearers.

### Background of the Invention

An inflammatory condition called stomatitis prothetica is a common complaint in users of dentures. According to Budtz-Jörgensen, E. (Community Dent. Oral Epidemiol., 1975, 3: 115-119), the frequency is 65% among elderly users of dentures.

Stomatitis prothetica is the designation for the mucosal inflammations frequently observed in users of dentures, particularly in those parts of the mouth coming into contact with the denture. The condition may occur as a simple inflammation, restricted to a few parts, or it may be spread over the whole area in contact with the denture. The condition commonly occurs underneath the dental plate covering the hard palate, where it may cause swelling.

Sometimes the inflammation spreads to the lips and corners of the mouth, where it may give rise to stinging and burning (cheilosis angularis).

In most cases, stomatitis prothetica is caused by a fungal infection, normally Candida albicans, (thrush, a yeast-like fungus) (Budtz-Jörgensen, E., Community Dent. Oral Epidemiol., 1975, 3: 115-119). Microscopic examination of the denture and tissue samples from the palate clearly shows evidence of growth.

Clinical and histological evidence clearly shows that the condition is a hypersensitivity reaction to C. albicans, which is a species of yeast-like fungus normally occurring in adults. In this connection it should be noted that a reduction in the number of Candida colonies leads to a relative improvement in the condition.

It is clear that the treatment of stomatitis prothetica consists of the partial or complete removal of the fungal infection. Local antimycotic therapy is one way of achieving this.

A large number of medicaments designed for local treatment of fungal conditions in the oral cavity are available today.

One preparation is nystatin ointment, which is applied to the upper part of the dental plate fitting against the palate. The ointment should be applied three times a day for one week. A powder form of nystatin for sprinkling on to the dentures has also been proposed.

Amphotericin B, another preparation for local treatment of fungalinfections, may be given as lozenges, to be taken 4 to 8 times daily.

Miconazole gel should be applied to the part of the denture fitting against the mucosa, 4 times a day for a fortnight. The denture should be inserted immediately after application, and any superfluous gel should be held in the mouth as long as possible before swallowing.

The above therapies are certainly effective but are very inconvenient to the patients. Moreover, the condition recurs soon after the course of treatment has been completed, because C. albicans is widely present in the human body. An antimycotic preparation with another type of administration is clearly needed to circumvent the above disadvantages.

U.S. Patent 3,476,854 discloses the treatment of dentures with liners and tissue conditioners, consisting of plastic compositions containing resins selected from lower alkyl methacrylates, silicone, vinyl co-polymers and polyamide, in which an antifungal compound is dispersed. The antifungal agents are zinc and copper salts of monocarboxylic fatty acids, up to medium chain length fatty acids, benzoic acid, benzyl benzoate, salicylic acid and benzyl salicylate. The object of this composition is to provide a cushion for dentures in the form of a plastic liner for the conditioning of ill-fitting dentures. The liner is deposited in the form of a gel so that the resulting plastic layer has sufficient thickness to form a cushion. The liner contains an antifungal preservative, which preservative is specifically selected so as not to be leached from the liner. Thus the liner does not constitute a drug delivery system.

PCT Application WO 87/02580, EP 298271 and DE 3,720,147 disclose the treatment of onychomycosis with a nail coating composition containing a film-forming, water-insoluble material and an antimycotic agent. The film forming material is e.g. polyvinylacetate, mixed polymers of vinyl acetate and acrylic or crotonic acid, polymers of fatty vinyl esters or methacrylate polymers. The antimycotic agent is tioconazole, econazole, miconazole, ketoconazole or clotrimazole. In all three cases, the object is to form a dry drug delivery system, allowing the antimycotic agent to be absorbed directly in a dry or undissolved state into the nail from the nail coating. The effect of such a delivery system is normally considered insignificant. The composition according to WO 87/02580 may comprise chemical agents for modifying the physical and/or chemical properties of the vehicles and films formed from said vehicles. Examples of such agents are humidity controlling and tack controlling agents. Furthermore, the reference lacks a teaching of any hydrophilic plasticizer.

GB-A-1603899 discloses a polishing preparation or body tissue lotion comprising a methyl cellulose solution that may further contain a fungicide. Methyl cellulose is water-soluble and acts as a moisture binding agent. The preparation will liberate the fungicide quickly and cannot provide a sustained drug delivery.

EP-A2-250187 describes a medicament containing an extruded single or multilayered self-supporting thin film that is capable of adhering to a wet mucous surface and that contains a hydroxypropylcellulose which is water soluble and provides for the adhesion to wet surfaces. Furthermore, the film contains a homopolymer of ethylene oxide, a water insoluble polymer selected from the group consisting of ethyl cellulose, propyl cellulose, polyethylene and polypropylene, and a plasticizer. The film does not contain a solvent and no solvent is used in the extrusion of the film. The dry film is unsuitable for use on dentures.

JP published patent application No. 60-228412 (A) discloses a topical composition for application on the skin comprising a film-forming agent, an antifungal agent, a solvent, and a plasticizer. Prior to the application on the skin the composition is diluted with 10 volumes of water per volume of the composition.

JP published unexamined application No. 63-146972 discloses a hydrophilic adhesive composition comprising a water- and alcohol-soluble polymer, an alcohol-soluble and water-insoluble polymer, a softening agent, and a solvent, optionally blended with, i.a. an antifungal agent. The composition is made into an adhesive sheet that can be pressed onto a carrier, such as a plastic film, or the composition can be applied in various ways directly to the carrier material that i.a. can be used as an adhesive plaster for the mucous membrane in the oral cavity and for the skin. The composition is completely unsuitable to apply on dentures.

EP 55396 discloses a method for treating fungal infections of the skin with a fungicide composition comprising 0.1 - 1% active ingredient, 2 - 10% spreading agent, 1 - 8% solubilizer and cellulose ether as a film forming agent. JP patent application 59-83867 discloses a similar method and composition with the active ingredient being clotrimazole. In these two disclosures, as well, the drug should penetrate from the film-forming layer directly into the skin.

EP-A1-0381446 discloses a sustained release antifungal varnish composition comprising, i.a., an antimycotic drug, a film-forming agent and a plasticizer. The citation falls under Article 54, paragraph 3, EPC.

### Summary of the Invention

The object of the present invention is to provide a drug delivery system having a long-term antimycotic effect in the oral cavity of denture wearers and which does not cause the patient major inconvenience.

The invention provides a denture lacquer which is characterized in that it comprises an antimycotic agent, a water-insoluble film-forming agent, from 1 to 25 wt.% of glycerol mono-oleate as a hydrophilic plasticizer and a solvent.

The lacquer may advantageously comprise from 1 to 30 wt.% of at least one antimycotic agent, preferably from 2 to 10 wt.%; from 1 to 20 wt.% of at least one water-insoluble film-forming agent, preferably from 5 to 15 wt.%; and preferably from 10 to 20 wt.% of glycerol mono-oleate as the hydrophilic plasticizer. The balance of the composition comprises at least one solvent and optional ingredients. Shellac is one optional ingredient, which may be present in an amount of from 0 to 10 wt.%, preferably from 0.5 to 5 wt.%. Shellac is useful for its adhesive properties.

The lacquer is such that when it is applied to an artificial denture, it forms a mechanically stable coating on the denture which provides for the extended release of antimycotic agent to the wearer's oral mucosa.

By "film-forming agent" is meant any compound which is capable of forming a film following deposition of a mixture of a vehicle and said agent on a surface, such as an artificial denture, and evaporation of the vehicle.

The invention further comprises a method for treating fungal infections in the mouth cavity of users of artificial dentures comprising applying the foregoing lacquer to artificial dentures. The lacquer is applied to the dentures by any convenient method. Typically, the lacquer should be applied by painting it onto the portion of the denture fitting against the mucosa. The lacquer should be permitted to dry thoroughly before insertion of the denture into the mouth of the user.

### Detailed Description of the Invention

The invention is based upon the fact that by application of a denture lacquer according to the invention to the surfaces in contact with the oral mucosa after the denture has been put into place, the antimycotic agent will be released to the aqueous environment in the oral cavity in therapeutically effective concentrations over a period of 2-3 weeks. This means that, with the present invention, patients will only need to treat their dentures with the denture lacquer at relatively long intervals.

The lacquer is adapted to form, once applied to the denture and dried, a stable and flexible coating on the denture which has advantageous mechanical properties. Moreover, the coating thus formed releases the antimycotic agent to the denture wearer's oral mucosa over an extended period of time, functioning as a thin drug-releasing matrix. The extended drug-release profile provides the wearer with continuous, extended protection from fungal growth in and around the oral mucosa, after only a single application of lacquer. The precise duration of continuous drug release, and the protection from fungal growth thus provided, may vary from one denture wearer to another. The duration over which the drug is released also depends upon the nature of the antimycotic agent, the nature of the other lacquer composition components, the relative amounts of those components, and the like. However, it has been found, according to the present invention, that release of antimycotic agent may advantageously proceed for one, two or possibly three weeks after a single application.

The water-insoluble film-forming agent may include, for example, water-insoluble derivatives of cellulose such as C₂-C₃ alkyl ethers of cellulose, e.g., ethyl- and propylcellulose; polyvinyl acetate; or (poly)acrylic acid derivatives, such as C₁-C₅ esters of acrylic or methacrylic acid. Shellac may be included as an optional further film-forming agent.

The solvent may comprise any suitable solvent capable of dissolving the water-insoluble film-former, such as, for example, lower alcohols (i.e., C₁-C₄ alcohols) such as methanol, ethanol, 1-propanol or 2-butanol, preferably ethanol; and esters of such lower alcohols with acetic acid, such as, for example, ethyl acetate, isopropyl acetate, or n-propyl acetate.

The plasticizer is a hydrophilic plasticizer, as opposed to a lipophilic plasticizer. Hydrophilic plasticizers provide a lacquer which has appropriate flexibility and mechanical stability. Moreover, it is believed that the use of a hydrophilic agent as the plasticizer favors the extended release of the antimycotic agent from the lacquer over time, once the lacquer is applied to the denture surface. Preferred hydrophilic plasticizers include, for example, glycerol, propanediol, polyethylene glycol (molecular weight 200 to 2000), glycerol mono-oleate, triethyl citrate, trimethyl citrate, C₂-C₂₆ monoglycerides, and glyceryl triacetate. Most preferably, the plasticizer comprises glycerol mono-oleate.

The relative content of hydrophilic plasticizer in the denture lacquer is important for the drug release from the denture coating to the saliva and oral mucosa. The greater the content of hydrophilic plasticizer the faster the drug release. The mechanical stability of the denture coating decreases, however, with increasing content of plasticizer and the mechanical stability is considered too low at plasticizer contents of above 25%. On the other hand a minimum content of 1% of plasticizer is necessary to prevent the denture coating from cracking.

The antimycotic agent employed should be stable and durable in the lacquer. Examples of such antimycotic agents include: miconazole, econazole, ketoconazole, tioconazole, and clotrimazole.

The effectiveness of the antimycotic preparation has been investigated in the following preliminary clinical studies:

### Eight-Patient Study

The following study was carried out in a hospital in Malmo, Sweden, with long-term geriatric patients during the period 12-28 September, 1988. Eight patients participated in the study. The patients were chosen according to a protocol, whose inclusion criterion was manifest stomatitis prothetica due to Candida infection, as diagnosed with "Oricult-N".

In Examination No. 1, all the patients exhibited a clinically moderate to pronounced redness of the oral mucosa. Substantial fungal growth (more than 100 colonies) was detected by means of "Oricult-N" yeast detection kit (Orion Diagnostica, Espoo, Finland). Denture lacquer prepared according to Example 1, below, was applied to the cleaned dentures. Five sets of dentures were cleaned with water; three sets were cleaned with hydrogen peroxide, alcohol and water. The dentures were allowed to dry for 1-6 hours before being replaced in the mouth.

Examination No. 2 was carried out one week later. There was a clinically reduced inflammation of the oral mucosa, and an objectively ascertained decrease in the number of colonies, verified with "Oricult-N". In one patient, who had worn his denture in the mouth about 90% of the time, the count for Candida colonies was completely negative.

Examination No. 3 was carried out one week subsequent to Examination No. 2. The number of colonies had increased over the number detected during Examination No. 2. Clinically, the inflammation was not as significant as Examination No. 1.

Examination No. 4 was carried out after yet another week. The result did not differ from that of Examination No. 3, and the number of colonies had not been affected by the cleaning of the dentures before the application.

### Five-Patient Study

In another clinical study, the participants were five female users of dentures with diagnosed oral Candida infection. The patients' dentures were painted with a denture lacquer prepared according to Example 1, below. The number of Candida colonies was counted both on the denture and on the adjacent oral mucosa, at intervals for four weeks. The results are set forth in Table 1:

**TABLE 1**

| NUMBER OF CANDIDA COLONIES | | | | | | | | |
|---|---|---|---|---|---|---|---|---|
| Patient No. | At the start | | At 1 week | | At 2 weeks* | | At 4 weeks | |
| | Denture | Mucosa | Denture | Mucosa | Denture | Mucosa | Denture | Mucosa |
| 1 | >100 | 28 | 0 | 0 | traces | 0 | >100 | 50 |
| 2 | >100 | >100 | 0 | 0 | 0 | 0 | VG | 55 |
| 3 | VG | abt. 100 | 0 | 0 | 0 | 0 | >100 | 25 |
| 4 | abt. 80 | abt. 70 | 0 | 0 | 0 | 0 | >100 | 60 |
| 5 | VG | VG | VG | VG | VG | 30 | >100 | 75 |
| VG = vigorous growth | | | | | | | | |

| | | | | | | | | |
|---|---|---|---|---|---|---|---|---|
| * = after this count, the dental lacquer was removed | | | | | | | | |

The first culture was carried out before application of the lacquer, and both denture and oral mucosa were tested for growth. The patients were asked to keep their dentures in place as much as possible and, if they removed them, to store the denture in humid conditions, although not immersed in a fluid.

At the start of the study, the count was positive for all patients, both from the dentures and from the oral mucosa. After treatment for one week, the count was negative for all patients, except patient number 5. In this patient, there was substantial fungal growth both on the mucosa and on the denture before the start of treatment. The oral mucosa showed a reduction in the number of colonies at 2 weeks' treatment.

At 2 weeks, the count was still negative in all the patients except No. 5. The lacquer was then removed and tested for residual concentration of miconazole.

After another fourteen days, there was a recurrence of Candida in the patients.

Table 2 shows the quantity of miconazole released from the dental lacquer during the same study.

**TABLE 2**

| Denture No. | Miconazole applied | Miconazole recovered | Miconazole released |
|---|---|---|---|
| 1 | 22.8 mg | 12.03 mg | 10.77 mg |
| 2 | 27.0 mg | 2.16 mg | 24.84 mg |
| 3 | 35.0 mg | 5.1 mg | 29.90 mg |
| 4 | 38.7 mg | 4.9 mg | 33.80 mg |
| 5 | 45.6 mg | 0.2 mg | 45.40 mg |

It may be concluded from these examinations that the lacquer has a definite therapeutic effect.

The following examples illustrate the preparation of different types of dental lacquers according to the invention, but should not be considered as limiting the scope of the invention.

### Example 1

5 g of miconazole is dissolved in 86 ml of ethanol, and 6 g of ethyl cellulose, 1 g of shellac, and 2 g of glycerol mono-oleate are added. The solution is stirred until completely dissolved. The lacquer appears as a semi-transparent, viscous solution.

The lacquer is dispensed from a glass bottle with a brush in the lid.

### Example 2

A lacquer is prepared as in Example 1, but using the same amount of ketoconazole in place of miconazole as the active ingredient.

### Example 3

4 g of econazole is dissolved in 33 ml of isopropanol and 33 ml of ethyl acetate. To this is added 2 g of glycerol mono-oleate and 28 g of polyvinyl acetate. The solution was stirred until completely dissolved.

## Claims (Claims for the following Contracting State(s): AT, BE, CH, DE, DK, FR, GB, IT, LI, LU, NL, SE)

1. A denture lacquer for the treatment of fungal infections in the oral cavity of users of artificial dentures, **characterized** in that it comprises an antimycotic agent, a water-insoluble film-forming agent, from 1 to 25 wt.% of glycerol mono-oleate as a hydrophilic plasticizer, and a solvent.

2. A denture lacquer according to claim 1, comprising from 1 to 30 wt.% of an antimycotic agent and from 1 to 30 wt.% of a water-insoluble film-forming agent.

3. A denture lacquer according to claim 2, comprising from 2 to 10 wt.% of an antimycotic agent, from 5 to 15 wt.% of a water insoluble film-forming agent, from 10 to 20 wt.% of glycerol mono-oleate as the hydrophilic plasticizer.

4. A denture lacquer according to any of claims 1-3, in which the antimycotic agent is selected from the group consisting of miconazole, ketoconazole, econazole, tioconazole, clotrimazole, and combinations thereof.

5. A denture lacquer according to claim 4, wherein the antimycotic agent comprises miconazole.

6. A denture lacquer according to any of claims 1 - 5, in which the water-insoluble film-forming agent is selected from the group consisting of C₂ - C₃ alkyl ethers of cellulose, polyvinyl acetate, C₁ - C₅ esters of acrylic or methacrylic acid, and combinations thereof.

7. A denture lacquer according to claim 6, wherein the film-forming agent comprises ethyl cellulose.

8. A denture lacquer according to any of claims 1-7, in which the solvent comprises a C₁ - C₄ alcohol or an ester formed from a C₁-C₄ alcohol and acetic acid.

9. A denture lacquer according to claim 8, wherein the solvent comprises ethanol.

10. A denture lacquer according to any of claims 1 - 9, comprising shellac in the amount of from 0 to 10 wt.%.

11. A denture lacquer according to claim 10, comprising shellac in the amount of from 0.5 to 5 wt.%.

12. A denture lacquer according to claim 10 or 11, comprising a mixture of shellac and as a film-forming agent ethyl cellulose.

13. An antimycotic coating on an artificial denture formed by applying to said denture a denture lacquer according to any of the preceding claims.

14. The use of a composition according to claims 1 to 13 for the manufacture of a medicament for the treatment of fungal infections in the mouth cavity of users of artificial dentures.

## Claims (Claims for the following Contracting State(s): ES)

1. A process for the preparation of a denture lacquer for the treatment of fungal infections in the oral cavity of users of artificial dentures, wherein an antimycotic agent, a water-insoluble film-forming agent and from 1 to 25 wt% of glycerol mono-oleate as a hydrophilic plasticizer are mixed with a solvent.

2. A process according to claim 1 comprising from 1 to 30 wt% of an antimycotic agent and from 1 to 30 wt.% of a water-insoluble film-forming agent.

3. A process according to claim 2, comprising from 2 to 10 wt.% of an antimycotic agent, from 5 to 15 wt.% of a water insoluble film-forming agent and from 10 to 20 wt.% of glycerol mono-oleate as the hydrophilic plasticizer.

4. A process according to any one of claims 1 - 3, in which the antimycotic agent is selected from the group consisting of miconazole, ketoconazole, econazole, tioconazole, clotrimazole, and combinations thereof.

5. A process according to claim 4, wherein the antimycotic agent comprises miconazole.

6. A process according to any one of claims 1 - 5, in which the water-insoluble film-forming agent is selected from the group consisting of C₂-C₃ alkyl ethers of cellulose, polyvinyl acetate, C₁-C₅ esters of acrylic or methacrylic acid, and combinations thereof.

7. A process according to claim 6, wherein the film-forming agent comprises ethyl cellulose.

8. A process according to any one of claims 1 - 7, in which the solvent comprises a C₁-C₄ alcohol or an ester formed from a C₁-C₄ alcohol and acetic acid.

9. A process according to claim 8, wherein the solvent comprises ethanol.

10. A process according to any one of claims 1 - 9, comprising shellac in the amount of from 0 to 10 wt.%.

11. A process according to claim 10, comprising shellac in the amount of from 0.5 to 5 wt.%.

12. A process according to claim 10 or 11, comprising a mixture of shellac and as a film-forming agent ethyl cellulose.

13. A process of forming an antimycotic coating on an artificial denture formed by applying to said denture a denture lacquer prepared according to any of the preceding claims.

14. The use of a composition prepared according to claims 1 to 13 for the manufacture of a medicament for the treatment of fungal infections in the mouth cavity of users of artificial dentures.

## Patentansprüche (Patentansprüche für folgende(n) Vertragsstaat(en): AT, BE, CH, DE, DK, FR, GB, IT, LI, LU, NL, SE)

1. Zahnersatz-Lack zur Behandlung von Pilzinfektionen in der Mundhöhle von Benutzern künstlichen Zahnersatzes, dadurch gekennzeichnet, daß er ein antimykotisches Mittel, ein wasserunlösliches filmbildendes Mittel, von 1 bis 25 Gew.-% Glycerin-monooleat als hydrophilen Weichmacher und ein Lösungsmittel umfaßt.

2. Zahnersatz-Lack nach Anspruch 1, umfassend von 1 bis 30 Gew.-% eines antimykotischen Mittels und von 1 bis 30 Gew.-% eines wasserunlöslichen filmbildenden Mittels.

3. Zahnersatz-Lack nach Anspruch 2, umfassend von 2 bis 10 Gew.-% eines antimykotischen Mittels, von 5 bis 15 Gew.-% eines wasserunlöslichen filmbildenden Mittels, von 10 bis 20 Gew.-% Glycerin-monooleat als hydrophilen Weichmacher.

4. Zahnersatz-Lack nach irgendeinem der Ansprüche 1 - 3, worin das antimykotische Mittel aus der aus Miconazol, Ketoconazol, Econazol, Tioconazol, Clotrimazol und deren Kombinationen bestehenden Gruppe ausgewählt ist.

5. Zahnersatz-Lack nach Anspruch 4, worin das antimykotische Mittel Miconazol umfaßt.

6. Zahnersatz-Lack nach irgendeinem der Ansprüche 1 - 5, worin das wasserunlösliche filmbildende Mittel aus der aus C₂-C₃-Alkylethern von Cellulose, Polyvinylacetat, C₁-C₅-Estern von Acryl- oder Methacrylsäure und deren Kombinationen bestehenden Gruppe ausgewählt ist.

7. Zahnersatz-Lack nach Anspruch 6, worin das filmbildende Mittel Ethylcellulose umfaßt.

8. Zahnersatz-Lack nach irgendeinem der Ansprüche 1 - 7, worin das Lösungsmittel einen C₁-C₄-Alkohol oder einen aus einem C₁-C₄-Alkohol und Essigsäure gebildeten Ester umfaßt.

9. Zahnersatz-Lack nach Anspruch 8, worin das Lösungsmittel Ethanol umfaßt.

10. Zahnersatz-Lack nach irgendeinem der Ansprüche 1 - 9, umfassend Schellack in der Menge von 0 bis 10 Gew.-%.

11. Zahnersatz-Lack nach Anspruch 10, umfassend Schellack in der Menge von 0,5 bis 5 Gew.-%.

12. Zahnersatz-Lack nach Anspruch 10 oder 11, umfassend ein Gemisch aus Schellack und Ethylcellulose als filmbildendes Mittel.

13. Antimykotischer Überzug auf einem künstlichen Zahnersatz, gebildet durch Aufbringen eines Zahnersatz-Lacks gemäß irgendeinem der vorhergehenden Ansprüche auf den Zahnersatz.

14. Verwendung einer Zusammensetzung gemäß Ansprüchen 1 bis 13 zur Herstellung eines Medikaments zur Behandlung von Pilzinfektionen in der Mundhöhle von Benutzern künstlichen Zahnersatzes.

## Patentansprüche (Patentansprüche für folgende(n) Vertragsstaat(en): ES)

1. Verfahren zur Herstellung eines Zahnersatz-Lacks zur Behandlung von Pilzinfektionen in der Mundhöhle von Benutzern künstlichen Zahnersatzes, bei dem ein antimykotisches Mittel, ein wasserunlösliches filmbildendes Mittel und von 1 bis 25 Gew.-% Glycerin-monooleat als hydrophiler Weichmacher mit einem Lösungsmittel gemischt werden.

2. Verfahren nach Anspruch 1, umfassend von 1 bis 30 Gew.-% eines antimykotischen Mittels und von 1 bis 30 Gew.-% eines wasserunlöslichen filmbildenden Mittels.

3. Verfahren nach Anspruch 2, umfassend von 2 bis 10 Gew.-% eines antimykotischen Mittels, von 5 bis 15 Gew.-% eines wasserunlöslichen filmbildenden Mittels und von 10 bis 20 Gew.-% Glycerin-monooleat als hydrophilen Weichmacher.

4. Verfahren nach irgendeinem der Ansprüche 1 - 3, bei dem das antimykotische Mittel aus der aus Miconazol, Ketoconazol, Econazol, Tioconazol, Clotrimazol und deren Kombinationen bestehenden Gruppe ausgewählt wird.

5. Verfahren nach Anspruch 4, bei dem das antimykotische Mittel Miconazol umfaßt.

6. Verfahren nach irgendeinem der Ansprüche 1 - 5, bei dem das wasserunlösliche filmbildende Mittel aus der aus C₂-C₃-Alkylethern von Cellulose, Polyvinylacetat, C₁-C₅-Estern von Acryl- oder Methacrylsäure und deren Kombinationen bestehenden Gruppe ausgewählt wird.

7. Verfahren nach Anspruch 6, bei dem das filmbildende Mittel Ethylcellulose umfaßt.

8. Verfahren nach irgendeinem der Ansprüche 1 - 7, bei dem das Lösungsmittel einen C₁-C₄-Alkohol oder einen aus einem C₁-C₄-Alkohol und Essigsäure gebildeten Ester umfaßt.

9. Verfahren nach Anspruch 8, bei dem das Lösungsmittel Ethanol umfaßt.

10. Verfahren nach irgendeinem der Ansprüche 1 - 9, umfassend Schellack in der Menge von 0 bis 10 Gew.-%.

11. Verfahren nach Anspruch 10, umfassend Schellack in der Menge von 0,5 bis 5 Gew.-%.

12. Verfahren nach Anspruch 10 oder 11, umfassend ein Gemisch aus Schellack und Ethylcellulose als filmbildendes Mittel.

13. Verfahren zur Bildung eines antimykotischen Überzugs auf einem künstlichen Zahnersatz, gebildet durch Aufbringen eines gemäß irgendeinem der vorhergehenden Ansprüche hergestellten Zahnersatz-Lacks auf den Zahnersatz.

14. Verwendung einer Zusammensetzung, hergestellt gemäß Ansprüchen 1 bis 13, zur Herstellung eines Medikaments zur Behandlung von Pilzinfektionen in der Mundhöhle von Benutzem künstlichen Zahnersatzes.

## Revendications (Revendications pour l'(les) Etat(s) contractant(s) suivant(s): AT, BE, CH, DE, DK, FR, GB, IT, LI, LU, NL, SE)

1. Vernis pour denture, pour le traitement d'infections fongiques dans la cavité buccale de personnes utilisatrices d'appareils dentaires ou de dentures artificielles, vernis caractérisé en ce qu'il comprend un agent antimycose, un agent formant une pellicule insoluble dans l'eau, de 1 à 25 % en poids de monooléate de glycérol comme plastifiant hydrophile, et un solvant.

2. Vernis pour denture selon la revendication 11 comprenant de 1 à 30 % en poids d'un agent antimycose et de 1 à 30 % en poids d'un agent formateur d'une pellicule insoluble dans l'eau.

3. Vernis pour denture selon la revendication 2, comprenant de 2 à 10 % en poids d'un agent antimycose, de 5 à 15 % en poids d'un agent formateur d'une pellicule insoluble dans l'eau, de 10 à 20 % en poids de monooléate de glycérol à titre de plastifiant hydrophile.

4. Vernis pour denture selon l'une quelconque des revendications 1 à 3, dans lequel l'agent antimycose est choisi dans le groupe consistant en le miconazole, le cétoconazole, l'éconazole, le tioconazole, le clotrimazole et leurs combinaisons.

5. Vernis pour denture selon la revendication 4, dans lequel l'agent antimycose est ou comprend du miconazole.

6. Vernis pour denture selon l'une quelconque des revendications 1 à 5, dans lequel l'agent formateur d'une pellicule insoluble dans l'eau est choisi dans l'ensemble consistant en des éthers alkyliques en C₂ ou C₃ de la cellulose, en du poly(acétate de vinyle), en des esters en C₁ à C₅ de l'acide acrylique ou de l'acide méthacrylique, et parmi leurs combinaisons.

7. Vernis pour denture selon la revendication 6, dans lequel l'agent formateur d'une pellicule comprend de l'éthylcellulose.

8. Vernis pour denture selon l'une quelconque des revendications 1 à 7, dans lequel le solvant comprend un alcool en C₁ à C₄ ou un ester formé à partir d'un alcool en C₁ à C₄ et de l'acide acétique.

9. Vernis pour denture selon la revendication 8, dans lequel le solvant ou comprend de l'éthanol.

10. Vernis pour denture selon l'une quelconque des revendications 1 à 9, comprenant de la gomme laque présente en une quantité de 0 à 10 % en poids.

11. Vernis pour denture selon la revendication 10, comprenant de la gomme laque présente en une quantité de 0,5 à 5 % en poids.

12. Vernis pour denture selon la revendication 10 ou 11, comprenant un mélange de gomme laque et, à titre d'agent formateur d'une pellicule, d'éthycellulose.

13. Revêtement antimycose sur une denture artificielle ou un appareil dentaire, revêtement formé par application à ladite denture ou audit appareil dentaire d'un vernis pour denture selon l'une quelconque des revendications précédentes.

14. Utilisation d'une composition selon les revendications 1 à 13 pour fabriquer un médicament pour le traitement d'infections fongiques dans la cavité buccale de personnes utilisatrices d'appareils dentaires ou des dentures artificielles.

## Revendications (Revendications pour l'(les) Etat(s) contractant(s) suivant(s): ES)

1. Procédé pour la préparation d'un vernis pour denture, pour le traitement d'infections fongiques dans la cavité buccale de personnes utilisatrices d'appareils dentaires ou de dentures artificielles, selon lequel on mélange un agent antimycose, un agent formateur d'une pellicule insoluble dans l'eau et de 1 à 25 % en poids de monooléate de glycérol, à titre de plastifiant hydrophile, avec un solvant.

2. Procédé selon la revendication 1, (dans lequel le vernis) comprend de 1 à 30 % en poids d'un agent antimycose et de 1 à 30 % en poids d'un agent formateur d'une pellicule insoluble dans l'eau.

3. Procédé selon la revendication 2, (dans lequel le vernis) comprend de 2 à 10 % en poids d'un agent antimycose, de 5 à 15 % en poids d'un agent formateur d'une pellicule insoluble dans l'eau et de 10 à 20 % en poids de monooléate de glycérol à titre de plastifiant hydrophile.

4. Procédé selon l'une quelconque des revendications 1 à 3, dans lequel l'agent antimycose est choisi dans le groupe consistant en le miconazole, le cétoconazole, l'éconazole, le tioconazole, le clotrimazole et leurs combinaisons.

5. Procédé selon la revendication 4, dans lequel l'agent antimycose est ou comprend du miconazole.

6. Procédé selon l'une quelconque des revendications 1 à 5, dans lequel l'agent formateur d'une pellicule insoluble dans l'eau est choisi dans le groupe consistant en des éthers alkyliques en C₂ ou C₃ de la cellulose, du poly(acétate de vinyle), des esters en C₁ à C₅ de l'acide acrylique ou de l'acide méthacrylique, et leurs combinaisons.

7. Procédé selon la revendication 6, dans lequel l'agent formateur de la pellicule est ou comprend de l'éthylcellulose.

8. Procédé selon l'une quelconque des revendications 1 à 7, dans lequel le solvant comprend un alcool en C₁ à C₄ ou un ester formé à partir d'un alcool en C₁ à C₄ et de l'acide acétique.

9. Procédé selon la revendication 8, dans lequel le solvant est ou comprend de l'éthanol.

10. Procédé selon l'une quelconque des revendications 1 à 9, comprenant (l'utilisation de) la gomme laque en une quantité de 0 à 10 % en poids.

11. Procédé selon la revendication 10, comprenant (l'utilisation de) la gomme laque en une quantité de 0,5 à 5 % en poids.

12. Procédé selon la revendication 10 ou 11, comprenant (l'utilisation d') un mélange de gomme laque et, comme agent formateur de pellicule, de l'éthycellulose.

13. Procédé pour former un revêtement antimycose sur un appareil dentaire ou denture, le revêtement étant formé par application, à ladite denture ou à l'appareil dentaire, d'un vernis pour denture préparé selon l'une quelconque des revendications précédentes.

14. Utilisation d'une composition, préparée selon les revendications 1 à 13 pour la fabrication d'un médicament pour le traitement d'infections fongiques dans la cavité buccale de personnes utilisatrices d'appareils dentaires ou des dentiers artificiels.
